# EUROPÄISCHE PATENTANMELDUNG

(11) **EP 3 159 326 A1**
(43) Veröffentlichungstag der Anmeldung: **26.04.2017**
(21) Anmeldenummer: 15191036.1
(22) Anmeldetag: 22.10.2015
(51) Int. Cl.: C07C 2/84, C07C 2/06, C07C 5/25, C10G 9/00, B01J 12/00

(54) **PROZESS UND ANLAGE ZUR HERSTELLUNG VON OLEFINEN**

(71) Anmelder: Linde Aktiengesellschaft, 80331 München (DE)
(72) Erfinder: Zimmermann, Heinz, 81476 München (DE)
(74) Vertreter: m patent group

(57) **Zusammenfassung**

Die Erfindung betrifft einen Prozess (100) zur Herstellung von Olefinen, bei dem Methan zusammen mit Sauerstoff einem katalytisch-thermischen Umsetzungsverfahren (10) zugeführt wird, das einen katalytischen Schritt (11, oxidative Kopplung von Methan, OCM) und einen sich dem katalytischen Schritt anschließenden thermischen Schritt (12, Post Bed Cracking) umfasst, wobei das Methan in dem katalytischen Schritt (11) zu einem Teil unter Bildung von Kohlenwasserstoffen mit zwei, drei und vier Kohlenstoffatomen umgesetzt wird, wodurch ein erstes Gasgemisch gebildet wird, das unter anderem die Kohlenwasserstoffe mit zwei, drei und vier Kohlenstoffatomen enthält, das erste Gasgemisch in dem thermischen Schritt (12) teilweise oder vollständig einem absteigenden Temperaturgradienten unterworfen wird, wodurch die Kohlenwasserstoffe mit zwei, drei und vier Kohlenstoffatomen zu einem Teil unter Bildung von Olefinen mit vier Kohlenstoffatomen, darunter 1-Buten, umgesetzt werden und ein zweites Gasgemisch gebildet wird, das unter anderem die Olefine mit vier Kohlenstoffatomen enthält, und aus dem zweiten Gasgemisch eine erste Fraktion abgetrennt wird, die das 1-Buten enthält und in den thermischen Schritt (12) zurückgeführt wird. Es ist vorgesehen, dass die erste Fraktion in dem thermischen Schritt (12) lediglich einem Teil des Temperaturgradienten unterworfen wird, so dass 1-Buten zu einem Teil zu 2-Buten isomerisiert, jedoch nicht oder zu weniger als 20% zu Kohlenwasserstoffen mit anderer Kohlenstoffzahl umgesetzt wird. Eine entsprechende Anlage ist ebenfalls Gegenstand der Erfindung.

## Beschreibung

Die Erfindung betrifft einen Prozess und eine Anlage zur Herstellung von Olefinen gemäß den jeweiligen Oberbegriffen der unabhängigen Patentansprüche.

### Stand der Technik

Verfahren zur Herstellung höherer Kohlenwasserstoffe aus Methan durch oxidative Methankopplung (engl. Oxidative Coupling of Methane, OCM) befinden sich derzeit in intensiver Entwicklung. Bei der oxidativen Methankopplung werden ein methanreicher und ein sauerstoffreicher Strom in einen Reaktor eingespeist, wo der Sauerstoff des sauerstoffreichen Stroms und ein Teil des Methans des methanreichen Stroms unter Bildung von Wasser und Nebenprodukten zu höheren Kohlenwasserstoffen, insbesondere dem typischen Zielprodukt Ethylen, reagieren.

Beispielsweise in der US 2015/0152025 A1 und der US 2015/0210610 A1 sind kombinierte katalytisch-thermische Umsetzungsverfahren bekannt, die die oxidative Methankopplung als katalytischen Schritt umfassen. Dem katalytischen Schritt schließt sich ein thermischer Schritt, das sogenannte "Post Bed Cracking" (nachfolgend auch als postkatalytisches Dampfspalten bezeichnet) an. Bei diesem postkatalytischen Dampfspalten durchläuft das in dem katalytischen Schritt gebildete Gasgemisch einen absteigenden Temperaturgradienten, so dass geeignete, in dem Gasgemisch enthaltene Komponenten unter Bedingungen umgesetzt werden, die jenen eines normalen Dampfspaltverfahrens ähneln. Insbesondere enthält das dem postkatalytischen Dampfspalten unterworfene Gasgemisch, wie erwähnt, Wasser.

Zu den beim Dampfspalten von Kohlenwasserstoffen verwendeten Bedingungen sei auf einschlägige Fachliteratur, beispielsweise den Artikel "Ethylene" in Ullmann's Encyclopedia of Industrial Chemistry, Onlineausgabe, 15. April 2007, DOI 10.1002/14356007.a10_045.pub2, verwiesen.

Dem postkatalytischen Dampfspalten können neben dem in dem katalytischen Schritt gebildeten Gasgemisch auch weitere Kohlenwasserstoffe zugeführt werden. In der erwähnten US 2015/0152025 A1 wird beispielsweise vorgeschlagen, dem postkatalytischen Dampfspalten Alkane mit zwei und mehr Kohlenstoffatomen zuzuführen. Diese können von außen zugeführt werden und beispielsweise die sogenannten "Natural Gas Liquids" (nachfolgend auch als Erdgaskondensate bezeichnet) umfassen. Auch in der US 2015/0210610 A1 ist offenbart, dem postkatalytischen Dampfspalten weitere Kohlenwasserstoffe zuzuführen. Hierbei wird Erdgas einer Extraktionseinheit zugeführt, die Kohlenwasserstoffe mit zwei und mehr Kohlenstoffatomen von leichteren Komponenten wie Methan trennt. Das Methan wird dem katalytischen Schritt zugeführt. Die Kohlenwasserstoffe mit zwei und mehr Kohlenstoffatomen werden weiter in unterschiedliche Fraktionen aufgetrennt, die teilweise dem postkatalytischen Dampfspalten zugeführt werden.

Aus den genannten Druckschriften ist auch die Rückführung von Fraktionen zum postkatalytischen Dampfspalten bekannt, die aus einem Gasgemisch gewonnen werden, das stromab des postkatalytischen Dampfspaltens vorliegt. Hierbei handelt es sich um Methan und Paraffine mit zwei Kohlenstoffatomen.

In den erläuterten Verfahren werden jedoch nicht immer die gewünschten Produkte gebildet. Die vorliegende Erfindung stellt sich daher die Aufgabe, entsprechende Verfahren zu verbessern und die eingesetzten Verbindungen besser zu nutzen.

### Offenbarung der Erfindung

Diese Aufgabe wird durch einen Prozess und eine Anlage zur Herstellung von Olefinen mit den Merkmalen der jeweiligen unabhängigen Patentansprüche gelöst. Ausgestaltungen sind jeweils Gegenstand der abhängigen Patentansprüche sowie der nachfolgenden Beschreibung.

Vor der Erläuterung der Merkmale und Vorteile der vorliegenden Erfindung werden deren Grundlagen und die verwendeten Begriffe erläutert.

Flüssige und gasförmige Gemische können im hier verwendeten Sprachgebrauch reich oder arm an einer oder mehreren Komponenten sein, wobei "reich" für einen Gehalt von wenigstens 50%, 75%, 90%, 95%, 99%, 99,5%, 99,9% oder 99,99% und "arm" für einen Gehalt von höchstens 50%, 25%, 10%, 5%, 1%, 0,1% oder 0,01% auf molarer, Gewichts- oder Volumenbasis stehen kann. Der Begriff "überwiegend" kann der Definition von "reich" entsprechen. Flüssige und gasförmige Gemische können im hier verwendeten Sprachgebrauch ferner angereichert oder abgereichert an einer oder mehreren Komponenten sein, wobei sich diese Begriffe auf einen entsprechenden Gehalt in einem Ausgangsgemisch beziehen, aus dem das flüssige oder gasförmige Gemisch erhalten wurde. Das flüssige oder gasförmige Strom ist "angereichert", wenn dieses zumindest den 1,1-fachen, 1,5-fachen, 2-fachen, 5-fachen, 10-fachen, 100-fachen oder 1.000-fachen Gehalt, "abgereichert", wenn es höchstens den 0,9-fachen, 0,5-fachen, 0,1-fachen, 0,01-fachen oder 0,001-fachen Gehalt einer entsprechenden Komponente, bezogen auf das Ausgangsgemisch, enthält.

Die vorliegende Anmeldung verwendet zur Charakterisierung von Drücken und Temperaturen die Begriffe "Druckniveau" und "Temperaturniveau", wodurch zum Ausdruck gebracht werden soll, dass entsprechende Drücke und Temperaturen in einer entsprechenden Anlage nicht in Form exakter Druck- bzw. Temperaturwerte verwendet werden müssen, um das erfinderische Konzept zu verwirklichen. Jedoch bewegen sich derartige Drücke und Temperaturen typischerweise in bestimmten Bereichen, die beispielsweise ± 1%, 5%, 10%, 20% oder sogar 50% um einen Mittelwert liegen. Entsprechende Druckniveaus und Temperaturniveaus können dabei in disjunkten Bereichen liegen oder in Bereichen, die einander überlappen. Insbesondere schließen beispielsweise Druckniveaus unvermeidliche oder zu erwartende Druckverluste, beispielsweise aufgrund von Abkühlungseffekten, ein. Entsprechendes gilt für Temperaturniveaus. Bei den hier in bar angegebenen Druckniveaus handelt es sich um Absolutdrücke.

Das "katalytisch-thermische Umsetzungsverfahren" der vorliegenden Erfindung umfasst einen katalytischen Schritt in Form einer oxidativen Methankopplung und einen sich dem katalytischen Schritt anschließenden thermischen Schritt in Form der eingangs erläuterten postkatalytischen Dampfspaltens. Beide Schritte können jeweils in einem oder mehreren Reaktoren bzw. Reaktoranordnungen durchgeführt werden, die parallel oder seriell angeordnet sein können. Immer schließt sich jedoch das postkatalytische Dampfspalten direkt dem katalytischen Schritt an. Insbesondere können der katalytische Schritt und das postkatalytische Dampfspalten in einem gemeinsamen Reaktor mit einer katalytischen Zone (mit einem Katalysatorbett) und einer postkatalytischen Zone (ohne Katalysatorbett) durchgeführt werden. Das postkatalytische Dampfspalten wird insbesondere unter Verwendung von Abwärme des katalytischen Schritts durchgeführt bzw. unter Verwendung von Wärme, die in dem in dem katalytischen Schritt gebildeten Gasgemisch enthalten ist.

Wie erwähnt durchläuft ein in dem katalytischen Schritt gebildetes Gasgemisch beim postkatalytischen Dampfspalten einen absteigenden Temperaturgradienten unterworfen. Dieser umfasst beispielsweise einen Temperaturbereich von 1.000 bis 700 °C, d.h. zu Beginn des postkatalytischen Dampfspaltens beträgt die Temperatur höchstens 1.000 °C, insbesondere 800 bis 1.000 °C, beispielsweise 880 bis 950 °C. Am Ende des postkatalytischen Dampfspaltens, d.h. am Austritt des verwendeten Reaktors, beträgt die Temperatur mindestens 700 °C, insbesondere 700 bis 900 °C, beispielsweise 750 bis 880 °C. In herkömmlichen Verfahren zum Dampfspalten können die verwendeten Spaltbedingungen unter anderem durch den Partialdruck der eingesetzten Kohlenwasserstoffe, der durch die Zugabe unterschiedlicher Dampfmengen und den verwendeten Druck eingestellt werden kann, die Verweildauer im Spaltofen sowie die in diesem verwendeten Temperaturen und Temperaturprofile beeinflusst werden. Auch die Geometrie und Gestaltung der verwendeten Reaktoren spielt eine Rolle.

Auch beim postkatalytischen Dampfspalten können diese Parameter, sofern sie nicht durch den katalytischen Schritt bzw. die dort geforderten Bedingungen festgelegt sind, beeinflusst werden. Ein bedeutender Einflussfaktor auf die Spaltbedingungen für zusätzlich eingespeiste Kohlenwasserstoffe ist hier jedoch die Einspeisestelle in den Reaktor. Diese legt fest, bei welcher Temperatur des erwähnten absteigenden Temperaturgradienten die Zuspeisung erfolgt und welchen Temperaturen die eingespeisten Kohlenwasserstoffe wie lange ausgesetzt werden. Das aus dem katalytischen Schritt stammende Gasgemisch durchläuft den Temperaturgradienten vollständig, wobei es einen entsprechenden Reaktor oder eine entsprechende Reaktorzone innerhalb einer definierten Zeit, die die Verweildauer bestimmt, durchströmt. Werden zusätzliche Kohlenwasserstoffe beispielsweise nach der Hälfte der Wegstrecke, die durch das Gasgemisch aus dem katalytischen Schritt durchlaufen wird, zugespeist, beträgt die Verweildauer die Hälfte und die Temperatur ist entsprechend dem Temperaturprofil des Temperaturgradienten reduziert. Nachfolgend ist davon die Rede, dass beim postkatalytischen Dampfspalten einer zugespeisten Komponente eine definierte Umsetzung erfolgt. Diese auf den ersten Blick als bloße Aufgabenstellung erscheinende Angabe stellt sich bei angemessener Betrachtungsweise jedoch als konkrete Anweisung an den Fachmann dar, der die Temperaturen, Verweildauern, die spezifische Geometrie und Gestaltung der verwendeten Reaktoren usw. kennt.

Wie oben erwähnt, beeinflussen eine Reihe von Parametern die Spaltbedingungen. Diese sind in jedem Reaktor unterschiedlich und unterscheiden sich je nach Verfahrensführung. Es ist daher nicht möglich, beispielsweise konkrete Angaben zu machen, beispielsweise welcher Temperatur oder Verweildauer ein entsprechender, zusätzlich eingespeister Kohlenwasserstoff beim postkatalytischen Dampfspalten genau ausgesetzt werden soll, da konkrete Werte immer auch von anderen verwendeten Parametern abhängen und keine absolute Gültigkeit besitzen. Die Verwendung einer konkreten Temperatur könnte, bei entsprechender Auslegung anderer Parameter, auch dazu führen, dass sich die im Rahmen der vorliegenden Erfindung erwünschten Effekte nicht einstellen.

Die Charakterisierung der Spaltbedingungen über die Bildung oder Umsetzung einzelner Komponenten ist beim Dampfspalten völlig üblich und beispielsweise in dem eingangs erwähnten Artikel "Ethylene" in Ullmann's Encyclopedia of Industrial Chemistry beschrieben. Unter anderem hat sich Begriff der "Spaltschärfe" (engl. Cracking Severity) durchgesetzt. Für flüssige Ofeneinsätze kann die Spaltschärfe über das Verhältnis von Propylen zu Ethylen (P/E) oder als das Verhältnis von Methan zu Propylen (M/P) im Spaltgas auf Gewichtsbasis (kg/kg) beschrieben werden. Für gasförmige Ofeneinsätze kann dagegen die Umsetzung bzw. Konversion einer jeweils betrachteten Komponente des Ofeneinsatzes als Maß der Spaltschärfe angegeben werden. Insbesondere für Kohlenwasserstoffe mit vier Kohlenstoffatomen ist eine Beschreibung der Spaltschärfe bzw. der Spaltbedingungen über die Umsetzung von Schlüsselkomponenten gut geeignet. Der Fachmann auf dem Gebiet des Dampfspaltens kennt die die Spaltbedingungen beeinflussenden Parameter und verstellt diese in zweckmäßiger Weise. Im Zweifelsfall führen ihn einfache Routinemessungen ohne unzumutbaren Aufwand zum gewünschten Ergebnis.

### Vorteile der Erfindung

Die vorliegende Erfindung geht von einem Prozess zur Herstellung von Olefinen aus, bei dem Methan zusammen mit Sauerstoff einem katalytischen-thermischen Umsetzungsverfahren zugeführt wird, das einen katalytischen Schritt und einen sich dem katalytischen Schritt anschließenden thermischen Schritt umfasst. Hierbei wird das Methan in dem katalytischen Schritt zu einem Teil unter Bildung von Kohlenwasserstoffen mit zwei, drei und vier Kohlenstoffatomen umgesetzt, wodurch ein erstes Gasgemisch gebildet wird, das unter anderem diese Kohlenwasserstoffe mit zwei, drei und vier Kohlenstoffatomen enthält. Das erste Gasgemisch enthält, wie bereits eingangs ausführlich erläutert, neben den genannten Kohlenwasserstoffen auch nicht umgesetztes Methan, Wasser sowie weitere Nebenprodukte wie beispielsweise Kohlenmonoxid und Kohlendioxid.

Das erste Gasgemisch wird in dem thermischen Schritt anschließend zu einem Teil oder vollständig einem absteigenden Temperaturgradienten unterworfen, wobei die Kohlenwasserstoffe mit zwei, drei und vier Kohlenstoffatomen zu einem Teil unter Bildung von Olefinen mit vier Kohlenstoffatomen, darunter 1-Buten, umgesetzt werden. Auf diese Weise wird ein zweites Gasgemisch gebildet, das unter anderem die Olefine mit vier Kohlenstoffatomen enthält. Dem thermischen Schritt können, wie ebenfalls ausführlich zuvor erläutert, beliebige weitere Kohlenwasserstoffe zugespeist werden.

Aus dem zweiten Gasgemisch, also dem stromab des katalytisch-thermischen Umsetzungsverfahrens insgesamt bzw. dessen thermischen Schritt vorliegenden Gasgemisch, wird anschließend eine erste Fraktion abgetrennt, die das 1-Buten enthält. Diese wird in den thermischen Schritt zurückgeführt. Wie nachfolgend erläutert, kann die erste Fraktion ausschließlich oder überwiegend das 1-Buten enthalten, es kann sich jedoch auch um eine Sammelfraktion handeln, die neben dem 1-Buten weitere Kohlenwasserstoffe, insbesondere weitere Kohlenwasserstoffe mit vier Kohlenstoffatomen, enthält. Zu weiteren Details und Merkmalen eines entsprechenden Verfahrens, von dem die vorliegende Erfindung ausgeht, sei auf die obigen Erläuterungen bzw. auf die zitierten Druckschriften ausführlich verwiesen.

Erfindungsgemäß ist nun vorgesehen, dass die erste Fraktion, also die Fraktion, die das 1-Buten enthält, in dem thermischen Schritt lediglich einem Teil des Temperaturgradienten unterworfen wird, so dass 1-Buten zu einem Teil zu 2-Buten isomerisiert, jedoch nicht oder zu weniger als 20%, insbesondere zu weniger als 10% oder weniger als 5% (auf Gewichtsbasis) zu Kohlenwasserstoffen mit anderer Kohlenstoffzahl umgesetzt wird. Bei der Isomeriserung kann sich abhängig von der Temperatur nahezu ein Gleichgewicht zwischen 1- und 2-Buten herstellen, gefolgt von der Spaltung der Komponenten bzw. deren Umsetzung zu Verbindungen derselben Kohlenstoffanzahl. Die Rückführung des 1-Butens dient im Rahmen der vorliegenden Erfindung nicht dazu, dieses im eigentlichen Sinne zu spalten, also in Kohlenwasserstoffe geringerer Kohlenstoffzahl umzusetzen. Der thermische Schritt bzw. der Teil des thermischen Schritts, dem das 1-Buten unterworfen wird, wird vielmehr dazu verwendet, 1-Buten, das aus den nachfolgend erläuterten Gründen in einem entsprechenden zweiten Gasgemisch unerwünscht ist, zu isomerisieren.

Wie bereits im Rahmen der zuvor getroffenen Definitionen erläutert, ist es im Rahmen der vorliegenden Erfindung nicht möglich, konkrete Werte für die Temperaturen anzugeben, denen das 1-Buten in den thermischen Schritt unterworfen wird. Diese richten sich, wie erläutert, unter anderem nach der Verweildauer, den (Partial-)Drücken und der Gestaltung des verwendeten Reaktors. Der Fachmann kennt diese Zusammenhänge und nimmt eine geeignete Auswahl entsprechender Parameter vor, um zu dem gewünschten Ergebnis zu gelangen. Wie erwähnt, sind im Zweifel lediglich einfache Optimierungsmessungen erforderlich, die keinen unzumutbaren Aufwand für den Fachmann darstellen. Die Spaltbedingungen, denen das 1-Buten in den thermischen Schritt unterworfen wird, sind jedoch besonders mild.

Durch den Einsatz des erfindungsgemäßen Verfahrens lässt sich insbesondere die Butadienausbeute in einem entsprechenden thermischen Schritt erhöhen, weil das zu 2-Buten isomerisierte 1-Buten sich besonders bevorzugt zu Butadien umsetzt. Die Isomerisierung und die Spaltung (d.h. bevorzugt die Umsetzung zu Butadien) erfolgen in dem Temperaturbereich sehr schnell und es erfolgt ein hoher Umsatz in einem einzigen Schritt. Dieses Ziel wird durch eine besonders milde Spaltung entsprechender rückgeführter Komponenten erzielt. Im Gegensatz zu bekannten Verfahren, wie sie eingangs erläutert wurden, bei denen die Hauptzielverbindung Ethylen ist, ist die Zielsetzung im Rahmen der vorliegenden Erfindung eine andere, nämlich auch die Herstellung von Butadien neben anderen Olefinen wie Ethylen und Propylen.

Wie zuvor erläutert, wird in einem einem katalytischen Schritt nachgeschalteten thermischen Schritt ein absteigender Temperaturgradient verwendet, der einen Temperaturbereich von 1.000 bis 700 °C umfasst. Bevorzugte Werte und Beispiele sind oben angegeben. Insbesondere ist der Temperaturbereich von 750 bis 850 °C für die Isomerisierung von 1-Buten und die anschließende Umsetzung zu Butadien ("milde Spaltung") geeignet. Besonders vorteilhaft ist es, wenn die erste Fraktion in dem thermischen Schritt einer Temperatur von höchstens 800 °C ausgesetzt wird, also, wie erwähnt, die erste Fraktion den Temperaturgradienten nur zu einem Teil durchläuft, wodurch besonders milde Spaltbedingungen herbeigeführt werden. Die Verweildauer des ersten Gasgemischs in einem entsprechenden Reaktor liegt beispielsweise bei 500 bis 1.000 Millisekunden. Die erste Fraktion kann beispielsweise dem Temperaturgradienten bzw. dessen Teil für die Hälfte dieser Gesamtverweildauer ausgesetzt werden.

Wie bereits angesprochen, kann die erste Fraktion neben dem 1-Buten auch weitere Komponenten enthalten. Beispielsweise kann es sich um eine Fraktion aus ungesättigten Kohlenwasserstoffen handeln. Die erste Fraktion kann also neben dem 1-Buten überwiegend oder ausschließlich weitere Butene umfassen. Diese können auch Isobuten einschließen. Isobuten kann jedoch auch, weil es sich in dem thermischen Schritt ggf. schlecht umsetzen lässt, zuvor abgetrennt werden.

Es ist jedoch auch möglich, eine erste Fraktion derart zu bilden, dass sie überwiegend oder ausschließlich 1-Buten enthält. Das 1-Buten wird auf diese Weise gezielt einer Isomerisierung und anschließenden Bildung von Butadien unterworfen, so dass aufgrund der vergleichsweise geringen Recyclevolumina eine Kapazität des verwendeten Reaktors nicht überschritten wird.

Vorteilhafterweise wird aus dem zweiten Gasgemisch auch wenigstens eine weitere Fraktion abgetrennt und ebenfalls in den thermischen Schritt zurückgeführt, in dem thermischen Schritt jedoch einem größeren Teil des Temperaturgradienten unterworfen. Beispielsweise können Kohlenwasserstoffe mit zwei Kohlenstoffatomen, insbesondere entsprechende Paraffine, stromauf der ersten Fraktion in den thermischen Schritt zurückgeführt werden, so dass diese schärferen Spaltbedingungen unterworfen werden und sich auf diese Weise größere Mengen an Ethylen bilden.

Mit anderen Worten kann das zweite Gasgemisch Paraffine mit zwei, drei oder vier Kohlenstoffatomen enthalten und die wenigstens eine Fraktion eine solche Paraffine enthaltende Fraktion umfassen. Die Erfindung in der soeben erläuterten Ausführungsform ermöglicht damit die vollständige stoffliche Nutzung auch von entsprechenden Paraffinen.

In einem entsprechenden Prozess ist es weiter von Vorteil, wenn in dem zweiten Gasgemisch enthaltenes Kohlenmonoxid und/oder Kohlendoixid aus dem zweiten Gasgemisch abgetrennt, einer Methanisierung unterworfen, und dem katalytischthermischen Umsetzungsverfahren wieder zugeführt wird. Auf diese Weise lässt sich die Gesamtkohlenstoffausbeute eines entsprechenden Prozesses signifikant erhöhen.

Wie bereits erwähnt, eignet sich Isobuten aufgrund seiner Stabilität schlechter für die Bearbeitung durch ein Dampfspaltverfahren. Enthält das zweite Gasgemisch daher Isobuten, kann dieses aus dem zweiten Gasgemisch zu einem entsprechenden tert-Butylether, beispielsweise Methyl- oder Ethyl-tert-butylether, umgesetzt werden. Das Isobuten kann zuvor oder nach der Umsetzung zu dem Butylether aus dem Gasgemisch abgetrennt werden.

Im Rahmen der vorliegenden Erfindung werden, wie erwähnt, Olefine hergestellt, so dass das zweite Gasgemisch Ethylen, Propylen und/oder Butadien enthält, wobei aus dem zweiten Gasgemisch eine ethylenreiche, eine propylenreiche und/ eine butadienreiche Fraktion abgetrennt werden. Insbesondere eignet sich die vorliegende Erfindung, wie erwähnt, für die Herstellung von Butadien, weil im Rahmen der vorliegenden Erfindung in dem zweiten Gasgemisch enthaltenes 1-Buten in dem thermischen Schritt des katalytisch-thermischen Umsetzungsverfahrens zu 2-Buten isomerisiert und anschließend zu Butadien umgesetzt werden kann.

Im Rahmen der vorliegenden Erfindung kann das zweite Gasgemisch ferner Kohlenwasserstoffe mit fünf oder fünf und mehr Kohlenstoffatomen enthalten, wobei diese Kohlenwasserstoffe aus dem zweiten Gasgemisch unter Bildung einer Fraktion mit Kohlenwasserstoffen mit fünf und mehr Kohlenstoffatomen abgetrennt werden. Entsprechende Kohlenwasserstoffe umfassen beispielsweise Aromaten, die aus einer entsprechenden Fraktion rückgewonnen werden können.

Das dem katalytisch-thermischen Umsetzungsverfahren zugeführte Methan wird im Rahmen einer Ausführungsform der Erfindung teilweise oder vollständig aus Erdgas gewonnen, wobei das Methan in einem Abtrennverfahren aus dem Erdgas abgetrennt wird. Entsprechende Abtrennverfahren sind grundsätzlich, beispielsweise zur Gewinnung der eingangs erläuterten Erdgaskondensate, bekannt.

Im Rahmen einer bevorzugten Ausgestaltung der vorliegenden Erfindung wird in dem Abtrennverfahren zur Gewinnung des Methans ferner eine Fraktion gebildet, die Kohlenwasserstoffe mit zwei oder zwei und mehr Kohlenstoffatomen enthält. Diese Fraktion kann teilweise oder vollständig, beispielsweise auch nach Auftrennung in weitere Fraktionen, ebenfalls dem thermischen Schritt zugeführt werden.

Die vorliegende Erfindung erstreckt sich auch auf eine Anlage zur Durchführung eines Prozesses zur Herstellung von Olefinen, die dafür eingerichtet ist, Methan zusammen mit Sauerstoff einer Reaktoranordnung zuzuführen, die für ein katalytisch-thermisches Umsetzungsverfahren eingerichtet ist, das einen katalytischen Schritt und einen sich dem katalytischen Schritt anschließenden thermischen Schritt umfasst, wobei der katalytische Schritt dafür eingerichtet ist, das Methan zu einem Teil unter Bildung von Kohlenwasserstoffen mit zwei, drei und vier Kohlenstoffatomen umzusetzen, wodurch ein erstes Gasgemisch gebildet wird, das unter anderem diese Kohlenwasserstoffe mit zwei, drei und vier Kohlenstoffatomen enthält.

Der thermische Schritt ist dafür eingerichtet, das erste Gasgemisch zu einem Teil oder vollständig im absteigenden Temperaturgradienten zu unterwerfen, wobei die Kohlenwasserstoffe mit zwei, drei und vier Kohlenstoffatomen zu einem Teil unter Bildung von Olefinen mit vier Kohlenstoffatomen, darunter 1-Buten, umgesetzt werden, und ein zweites Gasgemisch gebildet wird, das unter anderem die Olefine mit vier Kohlenstoffatomen enthält. Schließlich sind Mittel vorgesehen, die dafür eingerichtet sind, aus dem zweiten Gasgemisch eine erste Fraktion abzutrennen, die das 1-Buten enthält, und diese erste Fraktion in den thermischen Schritt zurückzuführen.

Eine entsprechende Anlage ist erfindungsgemäß durch Mittel gekennzeichnet, die dafür eingerichtet sind, die erste Fraktion in dem thermischen Schritt lediglich einem Teil des Temperaturgradienten zu unterwerfen, so dass 1-Buten zu einem Teil zu 2-Buten isomerisiert, jedoch nicht oder zu weniger als 20% zu Kohlenwasserstoffen mit anderer Kohlenstoffzahl umgesetzt wird.

Eine entsprechende Anlage ist vorteilhafterweise zur Durchführung eines Verfahrens eingerichtet, wie es zuvor erläutert wurde. Auf die obigen Erläuterungen wird daher ausdrücklich verwiesen.

Die vorliegende Erfindung wird nachfolgend unter Bezugnahme auf die beigefügte Zeichnung näher erläutert, welche eine bevorzugte Ausführungsform der Erfindung in schematischer Darstellung zeigt.

### Kurze Beschreibung der Zeichnung

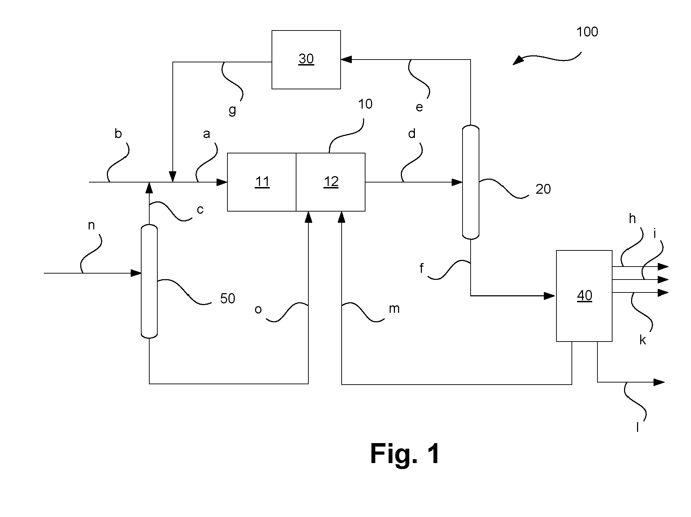

In Figur 1 ist ein Prozess gemäß einer Ausführungsform der Erfindung schematisch veranschaulicht.

### Ausführliche Beschreibung der Zeichnung

Figur 1 zeigt einen Prozess gemäß einer Ausführungsform der Erfindung, der insgesamt mit 100 bezeichnet ist, in einem vereinfachten, schematischen Ablaufplan. Zentrale Komponente des Prozesses 100 ist ein thermisches-katalytisches Umsetzungsverfahren 10, das einen katalytischen Schritt 11 und einen thermischen Schritt 12 umfasst. Wie erwähnt, handelt es sich bei dem katalytischen Schritt 11 um einen Schritt zur oxidativen Kopplung von Methan, bei dem thermischen Schritt 12 um einen postkatalytischen Dampfspaltschritt.

Dem thermisch-katalytischen Schritt 10 bzw. dessen katalytischem Schritt 11 wird ein Einsatzstrom a zugeführt, der aus einem sauerstoffreichen Strom b und einem methanreichen Strom c gebildet wird. In dem katalytischen Schritt 11 des katalytischthermischen Umsetzungsverfahrens 10 wird das Methan des Stroms c bzw. des Einsatzstroms a, wie erläutert, zu einem Teil unter Bildung von Kohlenwasserstoffen mit zwei, drei und vier Kohlenstoffatomen umgesetzt. Ein dabei gebildetes Gasgemisch, hier als "erstes" Gasgemisch bezeichnet, wird direkt dem sich anschließenden thermischen Schritt 12 des katalytisch-thermischen Umsetzungsverfahrens 10 zugeführt. Wie erwähnt, können der katalytische Schritt 11 und der thermische Schritt 12 des katalytisch-thermischen Umsetzungsverfahrens 10 in einem gemeinsamen Reaktor durchgeführt werden.

In dem katalytischen Schritt 12 des thermisch-katalytischen Umsetzungsverfahrens wird das erste Gasgemisch teilweise oder vollständig einem absteigenden Temperaturgradienten unterworfen, wodurch die Kohlenwasserstoffe mit zwei, drei und vier Kohlenstoffatomen aus dem ersten Gasgemisch zu einem Teil unter Bildung von Olefinen mit vier Kohlenstoffatomen, darunter 1-Buten, umgesetzt werden. In dem thermischen Schritt 12 des katalytisch-thermischen Umsetzungsverfahrens 10 wird auf diese Weise ein weiteres Gasgemisch, hier als "zweites" Gasgemisch bezeichnet, gebildet, das unter anderem die Olefine mit vier Kohlenstoffatomen enthält. Dieses zweite Gasgemisch wird in Form des Stroms d zunächst einem hier mit 20 bezeichneten Kohlendioxid- und/oder Kohlenmonoxidabtrennverfahren unterworfen, in welchem ein kohlendioxid- und/oder ein kohlenmonoxidreicher Strom e sowie ein kohlendioxid- und/oder kohlenmonoxidarmer Kohlenwasserstoffstrom f gebildet werden. Der kohlendioxid- und/oder kohlenmonoxidreiche Strom e kann insbesondere einem Methanisierungsverfahren 30 unterworfen werden, in dem Kohlendioxid und/oder Kohlenmonoxid zu Methan umgesetzt werden. Ein auf diese Weise erhaltener methanreicher Strom g kann dem Einsatzstrom a zugespeist werden.

Der kohlendioxid- und kohlenmonoxidarme Kohlenwasserstoffstrom f wird einem Trennverfahren 40 unterworfen, in welchem beispielsweise ein ethylenreicher Produktstrom h, ein propylenreicher Produktstrom i und ein butadienreicher Produktstrom k gebildet werden. Ferner wird in dem Trennverfahren 40 ein an Kohlenwasserstoffen mit fünf und mehr Kohlenstoffatomen reicher Strom I abgetrennt. Ein weiterer Strom m, der ebenfalls in dem Trennverfahren 40 abgetrennt wird, umfasst 1-Buten und ggf. weitere Kohlenwasserstoffe, wie zuvor erläutert, und wird dem thermischen Schritt 12 des katalytisch-thermischen Umsetzungsverfahrens zugeführt.

Wie erläutert, erfolgt die Zuführung des Stroms m in dem thermischen Schritt derart, dass der Strom m, zuvor auch als "erste Fraktion" bezeichnet, in dem thermischen Schritt lediglich einem Teil des dort verwendeten Temperaturgradienten unterworfen wird, so dass 1-Buten zu einem Teil zu 2-Buten isomerisiert, jedoch nicht oder zu weniger als 20% zu Kohlenwasserstoffen mit anderer Kohlenstoffzahl umgesetzt wird.

In Figur 1 ist ferner ein Abtrennverfahren 50 veranschaulicht, in welchem der bereits zuvor erwähnte methanreiche Strom c aus einem Erdgasstrom n abgetrennt wird. In dem Abtrennverfahren 50 fallen ferner ein oder mehrere weitere Ströme an, wie hier vereinfacht in Form des Stroms o veranschaulicht, die beispielsweise Kohlenwasserstoffe, insbesondere Paraffine, mit zwei oder zwei und mehr Kohlenstoffatomen, beispielsweise Erdgaskondensate, umfassen können. Ein oder mehrere solcher Ströme o können in den thermischen Schritt 12 des katalytischthermischen Umsetzungsverfahrens geführt und beispielsweise stromauf des Stroms m eingespeist werden.

## Patentansprüche

1. Prozess (100) zur Herstellung von Olefinen, bei dem Methan zusammen mit Sauerstoff einem katalytisch-thermischen Umsetzungsverfahren (10) zugeführt wird, das einen katalytischen Schritt (11) und einen sich dem katalytischen Schritt anschließenden thermischen Schritt (12) umfasst, wobei
- das Methan in dem katalytischen Schritt (11) zu einem Teil unter Bildung von Kohlenwasserstoffen mit zwei, drei und vier Kohlenstoffatomen umgesetzt wird, wodurch ein erstes Gasgemisch gebildet wird, das unter anderem die Kohlenwasserstoffe mit zwei, drei und vier Kohlenstoffatomen enthält,
- das erste Gasgemisch in dem thermischen Schritt (12) teilweise oder vollständig einem absteigenden Temperaturgradienten unterworfen wird, wodurch die Kohlenwasserstoffe mit zwei, drei und vier Kohlenstoffatomen zu einem Teil unter Bildung von Olefinen mit vier Kohlenstoffatomen, darunter 1-Buten, umgesetzt werden, und ein zweites Gasgemisch gebildet wird, das unter anderem die Olefine mit vier Kohlenstoffatomen enthält, und
- aus dem zweiten Gasgemisch eine erste Fraktion abgetrennt wird, die das 1-Buten enthält und in den thermischen Schritt (12) zurückgeführt wird,
**dadurch gekennzeichnet, dass** die erste Fraktion in dem thermischen Schritt (12) lediglich einem Teil des Temperaturgradienten unterworfen wird, so dass 1-Buten zu einem Teil zu 2-Buten isomerisiert, jedoch nicht oder zu weniger als 20% zu Kohlenwasserstoffen mit anderer Kohlenstoffzahl umgesetzt wird.

2. Prozess (100) nach Anspruch 1, bei dem der Temperaturgradient einen Temperaturbereich von 1.000 °C bis 700 °C umfasst.

3. Prozess (100) nach Anspruch 1 oder 2, bei dem der Teil des Temperaturgradienten, dem die erste Fraktion in dem thermischen Schritt (12) unterworfen wird, einen Temperaturbereich von 750 bis 850 °C umfasst.

4. Prozess (100) nach einem der vorstehenden Ansprüche, bei dem die erste Fraktion neben dem 1-Buten überwiegend oder ausschließlich weitere Butene, insbesondere überwiegend oder ausschließlich lineare Butene, umfasst.

5. Prozess (100) nach einem der Ansprüche 1 bis 3, bei dem die erste Fraktion überwiegend oder ausschließlich 1-Buten enthält.

6. Prozess (100) nach einem der vorstehenden Ansprüche, bei dem aus dem zweiten Gasgemisch wenigstens eine weitere Fraktion abgetrennt wird und ebenfalls in den thermischen Schritt (12) zurückgeführt wird, in dem thermischen Schritt (12) jedoch einem größeren Teil des Temperaturgradienten unterworfen wird.

7. Prozess (100) nach Anspruch 6, bei dem das zweite Gasgemisch Paraffine mit zwei, drei und/oder vier Kohlenstoffatomen enthält, und bei dem die wenigstens eine weitere Fraktion eine solche Paraffine enthaltende Fraktion umfasst.

8. Prozess (100) nach einem der vorstehenden Ansprüche, bei dem das zweite Gasgemisch Kohlenmonoxid und/oder Kohlendioxid enthält, und bei dem das Kohlenmonoxid und/oder Kohlendioxid aus dem zweiten Gasgemisch abgetrennt, einer Methanisierung (40) unterworfen, und dem katalytisch-thermischen Umsetzungsverfahren (10) wieder zugeführt wird.

9. Prozess (100) nach einem der vorstehenden Ansprüche, bei dem das zweite Gasgemisch Isobuten enthält, und bei dem das Isobuten zu einem tert-Butylether umgesetzt wird.

10. Prozess (100) nach einem der vorstehenden Ansprüche, bei dem das zweite Gasgemisch Ethylen, Propylen und/oder Butadien enthält, und bei dem aus dem zweiten Gasgemisch eine ethylenreiche, eine propylenreiche und/oder eine butadienreiche Fraktion abgetrennt werden.

11. Prozess (100) nach einem der vorstehenden Ansprüche, bei dem das zweite Gasgemisch Kohlenwasserstoffe mit fünf oder fünf und mehr Kohlenstoffatomen enthält, und bei dem aus dem zweiten Gasgemisch eine an Kohlenwasserstoffen mit fünf und mehr Kohlenstoffatomen reiche Fraktion abgetrennt wird.

12. Prozess (100) nach einem der vorstehenden Ansprüche, bei dem das dem katalytisch-thermischen Umsetzungsverfahren (10) zugeführte Methan zumindest zum Teil in einem Abtrennverfahren (50) aus Erdgas abgetrennt wird.

13. Prozess (100) nach Anspruch 12, bei dem in dem Abtrennverfahren (50) eine Fraktion gebildet wird, die Kohlenwasserstoffe mit zwei oder zwei und mehr Kohlenstoffatomen enthält, und bei dem diese Fraktion teilweise oder vollständig dem thermischen Schritt (12) zugeführt wird.

14. Anlage zur Durchführung eines Prozesses (100) zur Herstellung von Olefinen, die dafür eingerichtet ist, Methan zusammen mit Sauerstoff einer Reaktoranordnung zuzuführen, die für ein katalytisch-thermisches Umsetzungsverfahren (10) eingerichtet ist, das einen katalytischen Schritt (11) und einen sich dem katalytischen Schritt anschließenden thermischen Schritt (12) umfasst, wobei
- der katalytische Schritt (11) dafür eingerichtet ist, das Methan zu einem Teil unter Bildung von Kohlenwasserstoffen mit zwei, drei und vier Kohlenstoffatomen umzusetzen, wodurch ein erstes Gasgemisch gebildet wird, das unter anderem die Kohlenwasserstoffe mit zwei, drei und vier Kohlenstoffatomen enthält,
- der thermischen Schritt (12) dafür eingerichtet ist, das erste Gasgemisch teilweise oder vollständig einem absteigenden Temperaturgradienten zu unterwerfen, wodurch die Kohlenwasserstoffe mit zwei, drei und vier Kohlenstoffatomen zu einem Teil unter Bildung von Olefinen mit vier Kohlenstoffatomen, darunter 1-Buten, umgesetzt werden, und ein zweites Gasgemisch gebildet wird, das unter anderem die Olefine mit vier Kohlenstoffatomen enthält, und
- Mittel vorgesehen sind, die dafür eingerichtet sind, aus dem zweiten Gasgemisch eine erste Fraktion abzutrennen, die das 1-Buten enthält, und die erste Fraktion in den thermischen Schritt (12) zurückzuführen,
**gekennzeichnet durch** Mittel, die dafür eingerichtet sind, die erste Fraktion in dem thermischen Schritt (12) lediglich einem Teil des Temperaturgradienten zu unterwerfen, so dass 1-Buten zu einem Teil zu 2-Buten isomerisiert, jedoch nicht oder zu weniger als 20% zu Kohlenwasserstoffen mit anderer Kohlenstoffzahl umgesetzt wird.

15. Anlage nach Anspruch 14, die zur Durchführung eines Verfahrens nach einem der Ansprüche 1 bis 13 eingerichtet ist und hierzu entsprechende Mittel aufweist.
